# EUROPÄISCHE PATENTSCHRIFT

(11) **EP 4 585 720 B1**
(45) Veröffentlichungstag und Bekanntmachung des Hinweises auf die Patenterteilung: **10.06.2026**
(21) Anmeldenummer: 25150207.6
(22) Anmeldetag: 03.01.2025
(51) Int. Cl.: C25B 3/07, C25B 3/23, C25B 3/29, C25B 9/13, C25B 11/043, C25B 15/08, C07C 67/03

(54) **ELEKTROCHEMISCHES VERFAHREN ZUR HERSTELLUNG VON 1,2,3,4-BUTANTETRACARBONSÄURETETRAALKYLESTERN**
ELECTROCHEMICAL PROCESS FOR PRODUCING 1,2,3,4-BUTANETETRACARBOXYLIC ACID TETRAALKYL ESTERS
PROCÉDÉ ÉLECTROCHIMIQUE DE PRÉPARATION DE TÉTRAESTERS DE L'ACIDE 1,2,3,4-BUTANETÉTRACARBOXYLIQUE

(30) Priorität: 11.01.2024 EP 24151277
(43) Veröffentlichungstag der Anmeldung: 16.07.2025
(73) Patentinhaber: Evonik Oxeno GmbH & Co. KG, 45772 Marl (DE)
(72) Erfinder: Grass, Michael, 45721 Haltern am See (DE); Kraft, Johannes, 63067 Offenbach (DE); van Eickels, Michael, 45657 Recklinghausen (DE); Arndt, Sebastian, 65462 Ginsheim Gustavsburg (DE); Hoppe, Carl-Friedrich, 63584 Gründau (DE); Becker, Stephan, 45770 Marl (DE); Bremer, David Sascha, 45657 Recklinghausen (DE)
(74) Vertreter: Evonik Patent Association

(56) Entgegenhaltungen:
- EP-A2- 0 816 533
- EP-B1- 2 900 847
- WO-A1-02/42249
- WO-A1-2009/071478
- WO-A1-97/26389
- JP-A- H05 156 478
- US-A1- 2007 287 781

## Beschreibung

Die vorliegende Erfindung betrifft ein elektrochemisches Verfahren zur Herstellung von 1,2,3,4-Butantetracarbonsäuretetraalkylestern, die Alkylgruppen mit 1 bis 6 Kohlenstoffatomen enthalten. Das Verfahren umfasst die Elektrohydrodimerisierung von Dialkylmaleaten, die Alkylgruppen mit 1 bis 6 Kohlenstoffatomen enthalten, in einer einen Alkohol und ein Leitsalz enthaltenden Eduktlösung.

1,2,3,4-Butantetracarbonsäuretetraalkylester sind in der chemischen Industrie bekannte Ester und weisen die nachfolgende allgemeine Struktur auf 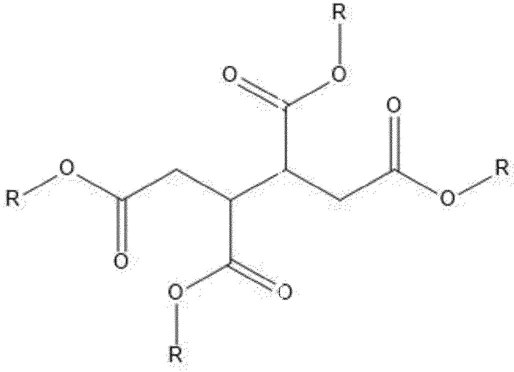 wobei alle vier Reste R jeweils für einen Alkylrest stehen. Diese Ester können beispielsweise als Weichmacher eingesetzt werden.

1,2,3,4-Butantetracarbonsäuretetraalkylester lassen sich grundsätzlich auf chemischem und elektrochemischem Wege herstellen. Die chemische Route verläuft über die Synthese von 1,2,3,4-Butantetracarbonsäure mit anschließender Veresterung mit einem Alkohol zum entsprechenden 1,2,3,4-Butantetracarbonsäuretetraalkylester. Die elektrochemische Route verläuft über eine Hydrodimerisierung von Dialkylmaleat, die an der Kathode stattfindet. Derartige Verfahren sind teilweise bereits in der Patentliteratur beschrieben worden, z. B. in der EP 0 816 533 A2, in der WO 97/26389 A1, in der WO 02/42249 A1 oder in der JP H05 156478 A1.

Die bekannten Verfahren weisen den Nachteil auf, dass sie großtechnisch entweder nicht wirtschaftlich oder nicht nachhaltig betrieben werden können. Weiterhin sollte eine alternative Route zur Herstellung der betreffenden 1,2,3,4-Butantetracarbonsäuretetraalkylester bereitgestellt werden.

Die Aufgabe der vorliegenden Erfindung war es deshalb, einen wirtschaftlichen und nachhaltigen Prozess zur Produktion von 1,2,3,4-Butantetracarbonsäuretetraalkylestern bereitzustellen. Die Aufgabe wird durch das in Anspruch 1 beschriebene Verfahren gelöst. Bevorzugte Ausführungsformen sind in den abhängigen Ansprüchen angegeben.

Das erfindungsgemäße Verfahren zur Herstellung von 1,2,3,4-Butantetracarbonsäuretetraalkylestern, die Alkylgruppen mit 1 bis 6 Kohlenstoffatomen, vorzugsweise Alkylgruppen mit 2 bis 5 Kohlenstoffatomen, besonders bevorzugt mit 5 Kohlenstoffatomen, enthalten, erfolgt durch Elektrohydrodimerisierung in mindestens einer Reaktionszone, die eine Anode und eine Kathode umfasst, mit einer Eduktlösung, die Dialkylmaleate, die Alkylgruppen mit 1 bis 6 Kohlenstoffatomen, vorzugsweise Alkylgruppen mit 2 bis 5 Kohlenstoffatomen enthalten, mindestens einen einwertigen Alkohol mit 1 bis 6 Kohlenstoffatomen, vorzugsweise mit 2 bis 5 Kohlenstoffatomen und ein Leitsalz umfassen, wobei an der Kathode die Dialkylmaleate zu 1,2,3,4-Butantetracarbonsäuretetraalkylestern elektrohydrodimerisiert und wobei als Anode und als Kathode bor-dotierte Diamantelektroden eingesetzt werden werden.

Der Einsatz von bor-dotierten Diamantelektroden als Elektrodenmaterial hat den Vorteil, dass die Zellspannung während der Reaktion geringer ist als bei Einsatz von bekannten Elektrodenmaterialien wie Graphit oder Glaskohlenstoff. Der elektrische Energieverbrauch einer Elektrolyse ist proportional zur Zellspannung. Mit bor-dotierten Diamantelektroden lässt sich das erfindungsgemäße Verfahren also energiesparender durchführen.

Die bei der Elektrohydrodimerisierung eingesetzten Dialkylmaleate, die Alkylgruppen mit 1 bis 6 Kohlenstoffatomen, vorzugsweise Alkylgruppen mit 2 bis 5 Kohlenstoffatomen, besonders bevorzugt mit 5 Kohlenstoffatomen, enthalten, werden nach dem bekannten Mechanismus umgesetzt. Die eingesetzten Dialkylmaleate sind großtechnisch erhältlich.

In einer bevorzugten Ausführungsform der vorliegenden Erfindung werden Dialkylmaleate, die jeweils Alkylgruppen mit 5 Kohlenstoffatomen aufweisen, d. h. Dipentylmaleate eingesetzt. Beim erfindungsgemäßen Verfahren entstehen dadurch 1,2,3,4-Butantetracarbonsäuretetraalkylester, die Alkylgruppen mit jeweils 5 Kohlenstoffatomen aufweisen, also ein 1,2,3,4-Butantetracarbonsäuretetrapentylester.

Der Begriff "pentyl" meint in diesem Zusammenhang, dass die erfindungsgemäßen Ester die verschiedenen Pentyl-Isomere n-Pentyl (1-, 2- oder 3-Pentyl), 2-Methylbutyl, 3-Methylbutyl, 2-Methylbut-2-yl, 3-Methylbut-2-yl, 2,2-Dimethylpropyl, insbesondere 2-Methylbutyl und/oder 3-Methylbutyl und/oder n-Pentyl, enthalten können. Der Begriff "pentyl" weist somit grundsätzlich nicht auf eine einzige spezielle C5-Alkylgruppe hin. Die erfindungsgemäß bevorzugt hergestellten 1,2,3,4-Butantetracarbonsäuretetrapentylester können demzufolge ausschließlich 2-Methylbutylgruppen, ausschließlich 3-Methylbutylgruppen, ausschließlich n-Pentylgruppen oder eine Mischung aus 2-Methylbutyl und/oder n-Pentyl und/oder 3-Methylbutylgruppen enthalten.

Die Eduktlösung umfasst neben dem Dialkylmaleat mindestens einen einwertigen Alkohol mit 1 bis 6 Kohlenstoffatomen, vorzugsweise mit 1 bis 5 Kohlenstoffatomen. Bevorzugte Alkohole sind Methanol und Pentanol. Das Pentanol kann dabei eine Mischung verschiedener isomerer Pentanole sein, beispielsweise eine Mischung aus 2-Methylbutanol und/oder 3-Methylbutanol und/oder 1-Pentanol. Der Alkohol fungiert bei der erfindungsgemäßen Elektrohydrodimerisierung als Lösemittel, weil in dem eingesetzten Alkohol das Dialkylmaleat und das Leitsalz gelöst werden können. Zeitgleich kann der Alkohol auch als Edukt für die Anodenreaktion dienen. Grundsätzlich kann auch eine Mischung von zwei oder mehr einwertigen Alkoholen mit unterschiedlicher Kohlenstoffkettenlänge eingesetzt werden. Dadurch können Mischester entstehen, die unterschiedliche Alkylreste enthalten. Es ist jedoch bevorzugt, dass nur ein Alkohol eingesetzt wird. Dieser eine Alkohol kann jedoch auch eine Mischung aus verschiedenen Isomeren mit gleicher Anzahl an Kohlenstoffatomen darstellen, wie es bereits beschrieben worden ist.

Es ist weiterhin grundsätzlich denkbar, dass das eingesetzte Dialkylmaleat und der eingesetzte Alkohol unterschiedliche Alkylgruppen mit unterschiedlicher Anzahl an Kohlenstoffatomen aufweisen. Erfindungsgemäß bevorzugt ist jedoch, dass die Anzahl der Kohlenstoffatome der Alkylgruppen des Dialkylmaleats und die Anzahl der Kohlenstoffatome des einwertigen Alkohols identisch sind . Soll also ein 1,2,3,4-Butantetracarbonsäuretetrapentylester aus Dipentylmaleaten hergestellt werden, wird als Lösemittel Pentanol eingesetzt.

Ein Einflussfaktor bei der erfindungsgemäßen Elektrohydrodimerisierung ist die Konzentration an Dialkylmaleat in Bezug zur Menge des Alkohols. Eine höhere Konzentration an Dialkylmaleat kann sich positiv auf die Ausbeute, die Selektivität und die Stromausbeute des zu bildenden 1,2,3,4-Butantetracarbonsäuretetraalkylester auswirken. In einer bevorzugten Ausführungsform der vorliegenden Erfindung beträgt die Konzentration an Dialkylmaleat 0,5 bis 4 mol / Liter einwertiger Alkohol, vorzugsweise 1 bis 3 mol / Liter einwertiger Alkohol.

Weiterhin umfasst die Eduktlösung, die bei der erfindungsgemäßen Elektrohydrodimerisierung eingesetzt wird, ein Leitsalz. Das Leitsalz sorgt während der elektrochemischen Reaktion für eine ausreichende Leitfähigkeit der Lösung. Grundsätzlich kann dabei jedes für die Eduktlösung und die Reaktion geeignete Leitsalz eingesetzt werden. Dem Fachmann sind entsprechende Leitsalze grundsätzlich bekannt.

Als Leitsalze können für die Elektrohydrodimerisierung insbesondere Leitsalze mit Tetralkylammonium-Kationen, Alkalimetall-Kationen und mit Anionen aus der Gruppe, bestehend aus aromatischsubstituierten Sulfonaten, Alkylsulfonaten, Acetaten, Perchloraten, Tetrafluoroboraten, Tetraphenylboraten, Bromiden, Iodiden, Phosphaten, Phosphonaten, Sulfaten, Alkylsulfaten, Hexafluorophosphaten, eingesetzt werden. Beispiele für geeignete Leitsalze sind Tetrabutylammonium-p-toluolsulfonat und Natriumacetat).

Auch die Konzentration des Leitsalzes kann ein Einflussfaktor bei der erfindungsgemäßen Elektrohydrodimerisierung sein. Insbesondere geringe Konzentrationen an Leitsalz wären an sich wirtschaftlich vorteilhaft, erhöhen aber die Zellspannung und wirken sich demnach negativ aus. Die Konzentration an Leitsalz beträgt im Rahmen der vorliegenden Erfindung vorzugsweise 0,05 bis 0,4 mol / Liter einwertiger Alkohol, vorzugsweise, 0,1 bis 0,4 mol/ Liter einwertiger Alkohol.

Die Eduktlösung muss zumindest das Dialkylmaleat, den einwertigen Alkohol und das Leitsalz enthalten. In einer bevorzugten Ausführungsform der vorliegenden Erfindung umfasst die Eduktlösung bei der erfindungsgemäßen Elektrohydrodimerisierung zusätzlich ein Colösemittel. Der Einsatz eines Colösemittels kann zu einem Absinken der Zellspannung führen. Geeignete Colösemittel reagieren bei der erfindungsgemäßen Elektrohydrodimerisierung nicht an der Kathode. Das Colösemittel wird vorzugsweise aus der Gruppe, bestehend aus Acetonitril, Dimethylsulfoxid, Tetrahydrofuran, Dioxan, Propylencarbonat, N,N-Dimethylformamid, organischen Carbonaten (z.B. Dimethylcarbonat), Dichlormethan, Chloroform und Aceton ausgewählt.

Die erfindungsgemäße Elektrohydrodimerisierung findet in einer geeigneten Reaktionszone statt, die einen oder mehrere Reaktoren umfassen kann. Da hier eine elektrochemische Reaktion stattfindet, muss ein Reaktor bekanntermaßen eine Anode und eine Kathode umfassen. Die Reaktoren können im Rahmen der vorliegenden Erfindung auch als Elektrolysezelle bezeichnet werden.

An der Kathode findet die gewünschte Umsetzung des Dialkylmaleats zum Zielprodukt, den 1,2,3,4-Butantetracarbonsäuretetraalkylestern, statt. Naturgemäß wird an der Anode gleichzeitig eine Oxidation ablaufen. Möglich ist beispielsweise, dass an der Anode der einwertige Alkohol zu einem Aldehyd oxidiert wird. Würde als einwertiger Alkohol Methanol eingesetzt, würde demnach Formaldehyd entstehen. Bei Einsatz von Butanol würde Butyraldehyd und bei Einsatz von Pentanol als einwertigem Alkohol würde Valeraldehyd entstehen. Besonders Valeraldehyd ist in der chemischen Industrie ein wichtiger Ausgangsstoff für Synthesen und stellt somit ein Wertprodukt da. Bei Einsatz von Pentanol würde es also zu einer Koppelproduktion von zwei unterschiedlichen Wertprodukten kommen, Valeraldehyd und 1,2,3,4-Butantetracarbonsäuretetrapentylester. Bei Einsatz von Dialkylmaleaten mit unterschiedlichen Alkylgruppen, wie z. B. Di(2-methylbutyl/n-pentyl)maleat in Pentanol, findet zusätzlich in geringem Umfang die Freisetzung von in diesem Fall 2-Methylbutanol, durch Austausch gegen Pentanol, und anschließende anodische Oxidation zu 2-Methylbutanal statt.

Im Rahmen der vorliegenden Erfindung werden als Anode und als Kathode bor-dotierte Diamantelektroden eingesetzt. Die Verwendung einer bor-dotierten Diamantelektrode als Kathode einer Hydrodimerisierung ist in WO 2009/071478 A1 offenbart.

Bei Einsatz von bor-dotierten Diamantelektroden kann der Abstand zwischen Anode und Kathode dabei vorzugsweise auf wenigstens 1 mm (mit einem geeigneten Kunststoff-Rahmen als Abstandshalter) eingestellt werden.

Die Anordnung von Anode und Kathode zueinander in der oder den Elektrolysezellen ist grundsätzlich nicht auf bestimme Anordnungen beschränkt. Klar ist, dass die Anordnung so gewählt wird, dass eine möglichst geringe Zellspannung auftritt. In einer bevorzugten Ausführungsform der vorliegenden Erfindung sind Anode und Kathode in der Elektrolysezelle planparallel zueinander angeordnet.

Die erfindungsgemäße Elektrohydrodimerisierung kann grundsätzlich als Batch-Prozess oder als kontinuierlicher Prozess ausgestaltet sein. Beim Batch-Prozess wird bekanntermaßen die Eduktlösung in den oder die Reaktor(en) bzw. in die Elektrolysezelle(n) eingefüllt, umgesetzt und die Produktmischung entnommen. Wird die Elektrohydrodimerisierung in kontinuierlicher Fahrweise betrieben, muss durchgehend frische Eduktlösung zum Reaktor bzw. zur Elektrolysezelle zudosiert werden und entstandene Produktlösung abgenommen werden.

Sowohl bei der Durchführung als Batch-Prozess als auch in kontinuierlicher Fahrweise gibt es einen kontinuierlichen Durchfluss durch den Reaktor bzw. durch die Elektrolysezelle. Dies dient u. a. dem Stofftransport hin zu den Elektroden oder von den Elektroden weg. Die Durchflussrate ist in diesem Zusammenhang das durch den Reaktor bzw. die Elektrolysezelle fließende Volumen pro Zeiteinheit.

Es ist grundsätzlich davon auszugehen, dass eine höhere Durchflussrate den Stofftransport an die Elektrode bzw. von der Elektrode weg verbessert. Die Durchflussrate liegt im Rahmen der vorliegenden Erfindung vorzugsweise im Bereich von 50 bis 700 l/h pro 100 cm² Elektrodenfläche bei planparalleler Anordnung, vorzugsweise von 100 bis 500 l/h pro 100 cm² Elektrodenfläche bei planparalleler Anordnung.

Eine möglichst hohe Temperatur ist vorteilhaft für eine niedrige Zellspannung, allerdings ergeben sich erhöhte Anforderungen an Werkstoffe und bei den kurzkettigen Alkoholen steigt der Dampfdruck stark an. Die einzustellende Temperatur ist deshalb ein Kompromiss zwischen diesen beiden Voraussetzungen. Es ist deshalb im Rahmen der vorliegenden Erfindung bevorzugt, dass die Elektrohydrodimerisierung bei einer Temperatur im Bereich von 20 bis 80 °C, vorzugsweise 25 bis 65 °C durchgeführt wird. Weiterhin ist es bevorzugt, dass die Elektrohydrodimerisierung bei einem Druck von 0,5 bis 3 bar, vorzugsweise 0,75 bis 2 bar durchgeführt wird.

Darüber hinaus sind die elektrochemischen Parameter wichtige Einflussfaktoren auf die Elektrohydrodimerisierung gemäß der vorliegenden Erfindung. Die Stromdichte beträgt bei der Elektrohydrodimerisierung vorzugsweise zwischen 1 und 25 mA/cm², vorzugsweise zwischen 2 bis 15 mA/cm², besonders bevorzugt zwischen 4 bis 10 mA/cm². Weiterhin ist es bevorzugt, dass lediglich eine stöchiometrische Ladungsmenge für die elektrochemische Reaktion zugeführt wird. Im vorliegenden Fall wird diese als elektrische Ladungsmenge pro mol Dialkylmaleat angegeben. Im Rahmen der vorliegenden Erfindung liegt die elektrische Ladungsmenge vorzugsweise im Bereich von 1 bis 1,5 F / mol Dialkylmaleat, vorzugsweise 1 bis 1,1 F / mol Dialkylmaleat. Ganz besonders bevorzugt sind nur stöchiometrische Ladungsmengen für die Reaktion erforderlich, d. h. die elektrische Ladungsmenge beträgt 1 F / mol Dialkylmaleat.

Um 1,2,3,4-Butantetracarbonsäuretetraalkylester zu erhalten, deren Alkylgruppen 2 oder mehr Kohlenstoffatome aufweisen, vorzugsweise deren Alkylgruppen 5 Kohlenstoffatome aufweisen, wäre es möglich, zunächst mittels des erfindungsgemäßen Verfahrens den jeweiligen1,2,3,4-Butantetracarbonsäuretetraalkylester aus Dimethylmaleat oder Diethylmaleat herzustellen und anschließend diesen Ester mit einem geeigneten Alkohol umzuestern.

Gegenstand der vorliegenden Erfindung ist deshalb auch ein Verfahren, bei dem durch Elektrohydrodimerisierung der 1,2,3,4-Butantetracarbonsäuretetramethylester oder 1,2,3,4-Butantetracarbonsäuretetraethylester hergestellt wird und anschließend der 1,2,3,4-Butantetracarbonsäuretetramethylester oder der 1,2,3,4-Butantetracarbonsäuretetraethylester mit mindestens einem einwertigen Alkohol mit 3 bis 6 Kohlenstoffatomen, vorzugsweise mit 5 Kohlenstoffatomen, zu 1,2,3,4-Butantetracarbonsäuretetraalkylestern, die Alkylgruppen mit 3 bis 6 Kohlenstoffatomen, vorzugsweise mit 5 Kohlenstoffatomen enthalten, umgeestert wird.

Die Umesterung ist ein an sich dem Fachmann bekanntes Verfahren, bei dem nach der vorliegenden Lehre ein längerkettiger Alkohol Methanol (bei Einsatz von Dimethylmaleat) oder Ethanol (bei Einsatz von Diethylmaleat) aus dem Ester verdrängt. Die Umesterung mit dem einwertigen Alkohol mit 2 bis 6 Kohlenstoffatomen, vorzugsweise mit 5 Kohlenstoffatomen wird vorzugsweise in Anwesenheit eines Katalysators oder mehrerer Katalysatoren, beispielsweise unter Verwendung von Brönstedt- oder LewisSäuren oder -Basen als Katalysator, durchgeführt. Als besonders geeignete Katalysatoren haben sich Schwefelsäure, Methansulfonsäure, p-Toluolsulfonsäure, Metalle oder deren Verbindungen erwiesen. Beispiele für besonders bevorzugte Metallkatalysatoren sind Zinnpulver, Zinn(II)oxid, Zinn(II)oxalat, Titansäureester wie Tetraisopropylorthotitanat oder Tetrabutylorthotitanat sowie Zirkoniumester wie Tetrabutylzirkonat sowie Natriummethanolat und Kaliummethanolat.

Die Umesterung kann in dem Fachmann bekannten, typischen Reaktoren unter üblichen Verfahrensbedingungen durchgeführt werden. Vorzugsweise findet das Verfahren bei Temperaturen bei oder oberhalb des Siedepunktes des bei der Reaktion gebildeten Alkohols statt, so dass dieser aus dem Reaktionsgemisch abdestilliert werden kann. Die Umesterung wird vorzugsweise bei einer Temperatur von 100 bis 300°C, bevorzugt bei 120 bis 270°C und insbesondere bei 140 bis 250°C durchgeführt. Der Druck innerhalb liegt vorzugsweise bei 0,1 bis 20 oder 15 bar, insbesondere bei 0,1 bis 10 bar.

Nachfolgend wird die vorliegende Erfindung anhand von Beispielen erläutert. Die dabei gezeigten speziellen Beispielausführungsformen sollen den Erfindungsgegenstand verdeutlichen, jedoch nicht beschränken.

### Beispiele

### Versuchsapparatur:

Zur Elektrohydrodimerisierung wurde eine Elektrolysezelle mit planparalleler Anordnung einer Anoden- und Kathodenplatte mit jeweils 100 cm² Fläche verwendet. Als Material für Anode und Kathode wurde entweder Glaskohlenstoff / Glaskohlenstoff, Graphit / Graphit oder Bor-dotierter Diamant / Bor-dotierter Diamant verwendet. Bei Einsatz von Glaskohlenstoff und Bor-dotiertem Diamant wurde ein PTFE-Abstandshalterrahmen mit 1 mm Dicke, bei Einsatz von Graphit ein PTFE-Abstandshalterrahmen mit 2 mm Dicke (bei 1 mm trat Kurzschlussverhalten auf und eine Elektrosynthese war nicht möglich) eingesetzt. Die Elektroden waren mit einer Stromversorgung und einem zusätzlichen Voltmeter verbunden.

Der Auslauf am oberen Bereich der Zelle wurde in eine Glaszwischenvorlage mit temperierbaren Außenmantel, der wiederum mit einem Thermostat verbunden war, geleitet. Der Ablauf der Zwischenvorlage war mit der Saugseite einer Peripheralradpumpe verbunden. Die Förderung in den Zulauf am unteren Bereich der Zelle erfolgte von der Druckseite der Pumpe.

### Beispiel 1 (nicht erfindungsgemäß):

Im Versuch wurde Glaskohlenstoff als Elektrodenmaterial verwendet. 74 g Di(2-methylbutyl/n-pentyl)maleat (289 mmol), 24 g (58 mmol) Tetrabutylammonium-p-toluolsulfonat wurden in 150 ml Pentanol gelöst und - nach Entnahme einer Probe - anschließend in die Zwischenvorlage der Versuchsapparatur gefüllt. Nach dem Starten der Pumpenzirkulation auf 280 I/h und Einstellung des Temperiermantels auf 50 °C wurde ein Strom von 600 mA (Stromdichte 6 mA/cm²) angelegt. Die Versuchsdauer betrug 13 Stunden. In diesem Zeitraum wurde eine elektrische Ladungsmenge von 0,29 F (entsprechend 1,01 F/mol Di(2-methylbutyl/n-pentyl)maleat) zugeführt. Die Zellspannung betrug zu Versuchsbeginn 7,2 V und stiegt bis zum Versuchsende auf 7,5 V an. Nach Versuchsende wurden 212 g Produktelektrolytlösung erhalten, die gaschromatographisch analysiert wurde. Diese enthielt 38,4 g Tetra(2-methylbutyl/n-pentyl)-1,2,3,4-butantetracarboxylat (Ausbeute 52%, Stromausbeute 51%), 13,3 g Di(2-methylbutyl/n-pentyl)succinat (Ausbeute 18%), weiterhin 20,7 g Di(2-methylbutyl/n-pentyl)maleat (entsprechend einem Umsatz von 72%).

### Beispiel 2 (nicht erfindungsgemäß):

Im Versuch wurde Graphit als Elektrodenmaterial verwendet. 74 g Di(2-methylbutyl/n-pentyl)maleat (289 mmol), 24 g (58 mmol) Tetrabutylammonium-p-toluolsulfonat wurden in 150 ml Pentanol gelöst und - nach Entnahme einer Probe - anschließend in die Zwischenvorlage der Versuchsapparatur gefüllt. Nach dem Starten der Pumpenzirkulation auf 280 I/h und Einstellung des Temperiermantels auf 50 °C wurde ein Strom von 600 mA (Stromdichte 6 mA/cm2) angelegt. Die Versuchsdauer betrug 13 Stunden. In diesem Zeitraum wurde eine elektrische Ladungsmenge von 0,29 F (entsprechend 1,01 F/mol Di(2-methylbutyl/n-pentyl)maleat) zugeführt. Die Zellspannung betrug zu Versuchsbeginn 9,6 V und sank bis zum Versuchsende auf 9,2 V. Nach Versuchsende wurden 211 g Produktelektrolytlösung erhalten, die gaschromatographisch analysiert wurde. Diese enthielt 27,5 g Tetra(2-methylbutyl/n-pentyl)-1,2,3,4-butantetracarboxylat (Ausbeute 37%, Stromausbeute 37%), 14,3 g Di(2-methylbutyl/n-pentyl)succinat (Ausbeute 19%), weiterhin 31,9 g Di(2-methylbutyl/n-pentyl)maleat (entsprechend einem Umsatz von 57%). Weiterhin enthielt die Lösung n-Pentanal (Valeraldehyd) und 2-Methylbutanal, die im Gegensatz zu den vorgenannten Kathodenprodukten Anodenprodukte darstellen.

### Beispiel 3 (nicht erfindungsgemäß):

Im Versuch wurde Graphit als Elektrodenmaterial verwendet. 74 g Di(2-methylbutyl/n-pentyl)maleat (289 mmol), 24 g (58 mmol) Tetrabutylammonium-p-toluolsulfonat wurden in 150 ml Pentanol und 27 ml Acetonitril aufgelöst und - nach Entnahme einer Probe - anschließend in die Zwischenvorlage der Versuchsapparatur gefüllt. Nach dem Starten der Pumpenzirkulation auf 280 I/h und Einstellung des Temperiermantels auf 50 °C wurde ein Strom von 600 mA (Stromdichte 6 mA/cm2) angelegt. Die Versuchsdauer betrug 13 Stunden. In diesem Zeitraum wurde eine elektrische Ladungsmenge von 0,29 F (entsprechend 1,01 F/mol Di(2-methylbutyl/n-pentyl)maleat) zugeführt. Die Zellspannung betrug zu Versuchsbeginn 5,8 V und stieg bis zum Versuchsende auf 6,6 V. Nach Versuchsende wurden 224 g Produktelektrolytlösung erhalten, die gaschromatographisch analysiert wurde. Diese enthielt 34,1 g Tetra(2-methylbutyl/n-pentyl)-1,2,3,4-butantetracarboxylat (Ausbeute 46%, Stromausbeute 46%), 12,6 g Di(2-methylbutyl/n-pentyl)succinat (Ausbeute 17%), weiterhin 17,3 g Di(2-methylbutyl/n-pentyl)maleat (entsprechend einem Umsatz von 77%).

### Beispiel 4 (erfindungsgemäß):

Im Versuch wurde Bor-dotierter Diamant als Elektrodenmaterial verwendet. 74 g Di(2-methylbutyl/n-pentyl)maleat (289 mmol), 24 g (58 mmol) Tetrabutylammonium-p-toluolsulfonat wurden in 150 ml Pentanol gelöst und - nach Entnahme einer Probe - anschließend in die Zwischenvorlage der Versuchsapparatur gefüllt. Nach dem Starten der Pumpenzirkulation auf 280 I/h und Einstellung des Temperiermantels auf 50 °C wurde ein Strom von 600 mA (Stromdichte 6 mA/cm2 ) angelegt. Die Versuchsdauer betrug 13 Stunden. In diesem Zeitraum wurde eine elektrische Ladungsmenge von 0,29 F (entsprechend 1,01 F/mol Di(2-methylbutyl/n-pentyl)maleat) zugeführt. Die Zellspannung betrug zu Versuchsbeginn 5,7 V und stiegt bis zum Versuchsende auf 6,2 V an. Nach Versuchsende wurden 210 g Produktelektrolytlösung erhalten, die gaschromatographisch analysiert wurde. Diese enthielt 35,6 g Tetra(2-methylbutyl/n-pentyl)-1,2,3,4-butantetracarboxylat (Ausbeute 48%, Stromausbeute 48 %), 14,9 g Di(2-methylbutyl/n-pentyl)succinat (Ausbeute 20 %), weiterhin 20,7 g Di(2-methylbutyl/n-pentyl)maleat (entsprechend einem Umsatz von 72%).

Die Ergebnisse der Ausführungsbeispiele 1 bis 4 werden in der nachfolgenden Tabelle 1 miteinander verglichen.

**Tabelle 1: Übersicht über die Ergebnisse der Beispiel 1 bis 4**

| Beispiel | 1 | 2 | 3 | 4** |
|---|---|---|---|---|
| Material Anode/Kathode | Glaskohlenstoff/ Glaskohlenstoff | Graphit/Graphit | Graphit/Graphit | Bor-dotiert Diamant/Bor-dotierter Diamant |
| Anodenfläche / cm² | 100 | 100 | 100 | 100 |
| Kathodenfläche (/ cm² | 100 | 100 | 100 | 100 |
| Elektrodenabstand / mm | 1 | 2 | 2 | 1 |
| Stromdichte / mA/cm² | 6,0 | 6,0 | 6,0 | 6,0 |
| Temperatur / °C | 50 | 50 | 50 | 50 |
| Zugabe Acetonitril / ml | - | - | 27 | - |
| Stromausbeute * / [%] | 51 | 37 | 46 | 48 |
| Durchschnitt Zellspannung über die gesamte Versuchsdauer / V | 7,35 | 9,4 | 6,2 | 5,95 |
| Elektrischer Energieverbrauch / kWh/t * | 1495 | 2646 | 1404 | 1291 |

| | | | | |
|---|---|---|---|---|
| * = Tetra(2-methylbutyl/n-pentyl)-1,2,3,4-butantetracarboxylat ** = erfindungsgemäß | | | | |

Der Übersicht in Tabelle 1 ist zu entnehmen, dass bei Einsatz von Bor-dotierten Diamantelektroden die geringste durchschnittliche Zellspannung erreicht wurde. Dadurch war der elektrische Energieverbrauch für die Elektrohydrodimerisierung im Vergleich mit allen anderen Versuchen am geringsten.

## Patentansprüche

1. Verfahren zur Herstellung von 1,2,3,4-Butantetracarbonsäuretetraalkylestern, die Alkylgruppen mit 1 bis 6 Kohlenstoffatomen enthalten, durch Elektrohydrodimerisierung in einer mindestens einer Reaktionszone, die eine Anode und eine Kathode umfasst, mit einer Eduktlösung, die Dialkylmaleate, die Alkylgruppen mit 1 bis 6 Kohlenstoffatomen enthalten, mindestens einen einwertigen Alkohol mit 1 bis 6 Kohlenstoffatomen und ein Leitsalz umfasst, wobei
an der Kathode die Dialkylmaleate zu 1,2,3,4-Butantetracarbonsäuretetraalkylestern elektrohydrodimerisiert werden, und
als Anode und als Kathode bor-dotierte Diamantelektroden eingesetzt werden.

2. Verfahren nach Anspruch 1, wobei die Anzahl der Kohlenstoffatome der Alkylgruppen des Dialkylmaleats und die Anzahl der Kohlenstoffatome des einwertigen Alkohols identisch ist.

3. Verfahren nach Anspruch 1 oder 2, wobei Leitsalze mit Tetralkylammonium-Kationen, Alkalimetall-Kationen und mit Anionen aus der Gruppe, bestehend aus aromatisch-substitutierten Sulfonaten, Alkylsulfonaten, Acetaten, Perchloraten, Tetrafluoroboraten, Tetraphenylboraten, Bromiden, Iodiden, Phosphaten, Phosphonaten, Sulfaten, Alkylsulfaten, Hexafluorophosphaten, eingesetzt werden.

4. Verfahren nach einem der vorhergehenden Ansprüche, wobei die 1,2,3,4-Butantetracarbonsäuretetraalkylester und die Dialkylmaleate jeweils Alkylgruppen mit 5 Kohlenstoffatomen aufweisen, vorzugsweise jeweils Alkylgruppen, die ausgewählt sind aus der Gruppe bestehend aus 2-Methylbutyl, 3-Methylbutyl und n-Pentyl

5. Verfahren nach einem der vorhergehenden Ansprüche, wobei als einwertiger Alkohol Methanol, Butanol oder ein Pentanol, vorzugsweise 1-Pentanol eingesetzt wird.

6. Verfahren nach einem der vorhergehenden Ansprüche, wobei bei der Elektrohydrodimerisierung zusätzlich ein Colösemittel eingesetzt wird, das aus der Gruppe, bestehend aus Acetonitril, Dimethylsulfoxid, Tetrahydrofuran, Dioxan, Propylencarbonat, N,N-Dimethylformamid, organischen Carbonaten, Dichlormethan, Chloroform und Aceton ausgewählt wird.

7. Verfahren nach einem der vorhergehenden Ansprüche, wobei die Elektrohydrodimerisierung bei einer Temperatur im Bereich von 20 bis 80 °C, vorzugsweise 25 bis 65 °C, durchgeführt wird.

8. Verfahren nach einem der vorhergehenden Ansprüche, wobei die die Elektrohydrodimerisierung bei einem Druck von 0,5 bis 3, vorzugsweise 0,75 bis 2 bar, durchgeführt wird.

9. Verfahren nach einem der vorhergehenden Ansprüche, wobei die Elektrohydrodimerisierung in einer Elektrolysezelle stattfindet und die Durchflussrate in der Elektrolysezelle im Bereich zwischen 50 und 700 l/h pro 100 cm² Elektrodenoberfläche, vorzugsweise zwischen 100 und 500 l/h pro 100 cm² Elektrodenfläche, beträgt.

10. Verfahren nach einem der vorhergehenden Ansprüche, wobei an der Anode während des Verfahrens der einwertige Alkohol mit 1 bis 6 Kohlenstoffatomen zu einem Aldehyd reagiert wird.

11. Verfahren nach Anspruch 10, wobei bei Einsatz von Pentanol als einwertigem Alkohol Valeraldehyd und zusätzlich 2-Methylbutanal entsteht.

12. Verfahren nach einem der vorhergehenden Ansprüche, wobei bei der Elektrohydrodimerisierung der 1,2,3,4-Butantetracarbonsäuretetramethylester hergestellt wird und anschließend der 1,2,3,4-Butantetracarbonsäuretetramethylester mit mindestens einem einwertigen Alkohol mit 2 bis 6 Kohlenstoffatomen zu 1,2,3,4-Butantetracarbonsäuretetraalkylestern, die Alkylgruppen mit 2 bis 6 Kohlenstoffatomen enthalten, umgeestert wird.

13. Verfahren nach einem der Ansprüche 1 bis 12, wobei bei der Elektrohydrodimerisierung der 1,2,3,4-Butantetracarbonsäuretetraethylester hergestellt wird und anschließend der 1,2,3,4-Butantetracarbonsäuretetraethylester mit mindestens einem einwertigen Alkohol mit 3 bis 6 Kohlenstoffatomen zu 1,2,3,4-Butantetracarbonsäuretetraalkylestern, die Alkylgruppen mit 3 bis 6 Kohlenstoffatomen enthalten, umgeestert wird.

14. Verfahren nach Anspruch 12 oder 13, wobei die Umesterung bei einer Temperatur von 100 bis 300°C, bevorzugt bei 120 bis 270°C durchgeführt wird.

## Claims

1. Process for producing tetraalkyl 1,2,3,4-butanetetracarboxylates containing alkyl groups having 1 to 6 carbon atoms by electrohydrodimerization in at least one reaction zone comprising an anode and a cathode with a reactant solution comprising dialkyl maleates containing alkyl groups having 1 to 6 carbon atoms, at least one monohydric alcohol having 1 to 6 carbon atoms and a conducting salt, wherein
the dialkyl maleates are electrohydrodimerized to afford tetraalkyl 1,2,3,4-butanetetracarboxylates at the cathode, and
the anode and the cathode employed are boron-doped diamond electrodes.

2. Process according to Claim 1, wherein the number of carbon atoms of the alkyl groups of the dialkyl maleate and the number of carbon atoms of the monohydric alcohol are identical.

3. Process according to Claim 1 or 2, wherein conducting salts having tetraalkylammonium cations, alkali metal cations and having anions from the group consisting of aromatic-substituted sulfonates, alkylsulfonates, acetates, perchlorates, tetrafluoroborates, tetraphenylborates, bromides, iodides, phosphates, phosphonates, sulfates, alkylsulfates, hexafluorophosphates are employed.

4. Process according to any of the preceding claims, wherein the tetraalkyl 1,2,3,4-butanetetracarboxylates and the dialkyl maleates each have alkyl groups having 5 carbon atoms, preferably each alkyl groups selected from the group consisting of 2-methylbutyl, 3-methylbutyl and n-pentyl.

5. Process according to any of the preceding claims, wherein the monohydric alcohol employed is methanol, butanol or a pentanol, preferably 1-pentanol.

6. Process according to any of the preceding claims, wherein the electrohydrodimerization additionally employs a cosolvent selected from the group consisting of acetonitrile, dimethyl sulfoxide, tetrahydrofuran, dioxane, propylene carbonate, N,N-dimethylformamide, organic carbonates, dichloromethane, chloroform and acetone.

7. Process according to any of the preceding claims, wherein the electrohydrodimerization is performed at a temperature in the range from 20°C to 80°C, preferably 25°C to 65°C.

8. Process according to any of the preceding claims, wherein the electrohydrodimerization is performed at a pressure of 0.5 to 3 bar, preferably 0.75 to 2 bar.

9. Process according to any of the preceding claims, wherein the electrohydrodimerization is carried out in an electrolysis cell and the flow rate in the electrolysis cell is in the range between 50 and 700 l/h per 100 cm² of electrode surface area, preferably between 100 and 500 l/h per 100 cm² of electrode surface area.

10. Process according to any of the preceding claims, wherein the monohydric alcohol having 1 to 6 carbon atoms is reacted to afford an aldehyde at the anode during the process.

11. Process according to Claim 10, wherein, when using pentanol as the monohydric alcohol, valeraldehyde and also 2-methylbutanal are formed.

12. Process according to any of the preceding claims, wherein the electrohydrodimerization produces the tetramethyl 1,2,3,4-butanetetracarboxylate and subsequently the tetramethyl 1,2,3,4-butanetetracarboxylate is transesterified with at least one monohydric alcohol having 2 to 6 carbon atoms to afford tetraalkyl 1,2,3,4-butanetetracarboxylates containing alkyl groups having 2 to 6 carbon atoms.

13. Process according to any of Claims 1 to 12, wherein the electrohydrodimerization produces the tetraethyl 1,2,3,4-butanetetracarboxylate and subsequently the tetraethyl 1,2,3,4-butanetetracarboxylate is transesterified with at least one monohydric alcohol having 3 to 6 carbon atoms to afford tetraalkyl 1,2,3,4-butanetetracarboxylates containing alkyl groups having 3 to 6 carbon atoms.

14. Process according to Claim 12 or 13, wherein the transesterification is performed at a temperature of 100°C to 300°C, preferably at 120°C to 270°C.

## Revendications

1. Procédé de production d'esters tétraalkyliques de l'acide 1,2,3,4-butanetétracarboxylique contenant des groupes alkyles ayant 1 à 6 atomes de carbone, par électrohydrodimérisation dans au moins une zone réactionnelle comprenant une anode et une cathode, avec une solution de départ comprenant des maléates de dialkyle contenant des groupes alkyles ayant 1 à 6 atomes de carbone, au moins un monoalcool ayant 1 à 6 atomes de carbone et un sel conducteur, dans lequel
au niveau de la cathode, les maléates de dialkyle sont électrohydrodimérisés en esters tétraalkyliques de l'acide 1,2,3,4-butanetétracarboxylique, et
des électrodes en diamant dopées au bore sont utilisées en tant qu'anode et en tant que cathode.

2. Procédé selon la revendication 1, dans lequel le nombre d'atomes de carbone des groupes alkyles du maléate de dialkyle et le nombre d'atomes de carbone du monoalcool sont identiques.

3. Procédé selon la revendication 1 ou 2, dans lequel on utilise des sels conducteurs comportant des cations tétraalkylammonium, des cations de métaux alcalins et des anions choisis dans le groupe constitué par des sulfonates à substitution aromatique, des alkylsulfonates, des acétates, des perchlorates, des tétrafluoroborates, des tétraphénylborates, des bromures, des iodures, des phosphates, phosphonates, des sulfates, des alkylsulfates, des hexafluorophosphates.

4. Procédé selon l'une des revendications précédentes, dans lequel les esters tétraalkyliques de l'acide 1,2,3,4-butanetétracarboxylique et les maléates de dialkyle comportent respectivement des groupes alkyles ayant 5 atomes de carbone, de préférence respectivement des groupes alkyles choisis dans le groupe constitué par le 2-méthylbutyle, le 3-méthylbutyle et n-pentyle.

5. Procédé selon l'une des revendications précédentes, dans lequel on utilise en tant que monoalcool le méthanol, le butanol ou le pentanol, de préférence le 1-pentanol.

6. Procédé selon l'une des revendications précédentes, dans lequel, lors de l'électrohydrodimérisation, on utilise en outre un cosolvant choisi dans le groupe constitué par l'acétonitrile, le diméthylsulfoxyde, le tétrahydrofurane, le dioxane, le carbonate de propylène, le N,N-diméthylformamide, des carbonates organiques, le dichlorométhane, le chloroforme et l'acétone.

7. Procédé selon l'une des revendications précédentes, dans lequel l'électrohydrodimérisation est effectuée à une température dans la plage de 20 à 80 °C, de préférence de 25 à 65 °C.

8. Procédé selon l'une des revendications précédentes, dans lequel l'électrohydrodimérisation est effectuée à une pression de 0,5 à 3 bars, de préférence de 0,75 à 2 bars.

9. Procédé selon l'une des revendications précédentes, dans lequel l'électrohydrodimérisation a lieu dans une cellule d'électrolyse et le débit dans la cellule d'électrolyse se situe dans la plage comprise entre 50 et 700 l/h pour 100 cm² de surface d'électrode, de préférence entre 100 et 500 l/h pour 100 cm² de surface d'électrode.

10. Procédé selon l'une des revendications précédentes, dans lequel, pendant le procédé, le monoalcool ayant 1 à 6 atomes de carbone est mis à réagir en un aldéhyde au niveau de l'anode.

11. Procédé selon la revendication 10, dans lequel, en cas d'utilisation de pentanol en tant que monoalcool, il se forme du valéraldéhyde et en complément, du 2-méthylbutanal.

12. Procédé selon l'une des revendications précédentes, dans lequel l'ester tétraméthylique de l'acide 1,2,3,4-butanetétracarboxylique est produit lors de l'électrohydrodimérisation, puis l'ester tétraméthylique de l'acide 1,2,3,4-butanetétracarboxylique subit une transestérification avec au moins un monoalcool ayant 2 à 6 atomes de carbone en des esters tétraalkyliques de l'acide 1,2,3,4-butanetétracarboxylique contenant des groupes alkyles ayant 2 à 6 atomes de carbone.

13. Procédé selon l'une des revendications 1 à 12, dans lequel l'ester tétraéthylique de l'acide 1,2,3,4-butanetétracarboxylique est produit lors de l'électrohydrodimérisation, puis l'ester tétraéthylique de l'acide 1,2,3,4-butanetétracarboxylique subit une transestérification avec au moins un monoalcool ayant 3 à 6 atomes de carbone en des esters tétraalkyliques de l'acide 1,2,3,4-butanetétracarboxylique contenant des groupes alkyles ayant 3 à 6 atomes de carbone.

14. Procédé selon la revendication 12 ou 13, dans lequel la transestérification est effectuée à une température de 100 à 300 °C, de préférence de 120 à 270 °C.
